# EUROPEAN PATENT SPECIFICATION

(11) **EP 3 790 467 B1**
(45) Date of publication and mention of the grant of the patent: **06.12.2023**
(21) Application number: 19799916.2
(22) Date of filing: 10.05.2019
(51) Int. Cl.: A61B 5/1455, A61B 5/0205, A61B 5/083, G16H 50/20, A61B 5/00, A61B 5/18, A61B 5/16, A61B 5/145

(54) **ABNORMAL BLOOD OXYGENATION LEVEL MONITORING SYSTEM AND SELF-MONITORING OXYGENATION SYSTEM**
SYSTEM ZUR ÜBERWACHUNG VON ANORMALEM BLUTSAUERSTOFFGEHALT UND SELBSTÜBERWACHENDES SAUERSTOFFGEHALTSSYSTEM
SYSTÈME DE SURVEILLANCE DE NIVEAU D'OXYGÉNATION SANGUINE ANORMALE, ET SYSTÈME D'OXYGÉNATION À AUTO-SURVEILLANCE

(30) Priority: 11.05.2018 US 201862670291 P
(43) Date of publication of application: 17.03.2021
(73) Proprietor: Spectronix Inc., Québec J9A 1L8 (CA)
(72) Inventor: ASSOUAD, Patrick, Ottawa, Ontario K1N 9K8 (CA)
(74) Representative: Fulton, David James
(86) International application number: PCT/CA2019/050637
(87) International publication number: WO 2019/213783

(56) References cited:
- EP-A1- 2 075 189
- WO-A2-2010/129528
- JP-A- H05 124 592
- US-A1- 2008 139 908
- US-A1- 2018 001 980
- US-B2- 6 671 529
- US-B2- 9 014 772

## Description

### FIELD OF THE DISCLOSURE

The present disclosure relates to blood oxygenation monitoring, and, in particular, to abnormal blood oxygenation level monitoring system and self-monitoring oxygenation system.

### BACKGROUND

Change in oxygen content and air pressure can have a great impact on the health of the human body. A range of human activities in hazardous environments, often requiring an oxygen providing apparatus or requiring being constrained in a sealed environment and/or pressurized environment, like deep water diving, flying a fighter plane, working at high altitudes or great depths (i.e. deep tunnel construction, etc.), or being within a hyperbaric chamber, may result in an abnormal blood oxygen level. A low blood oxygen level (hypoxia) may result in headaches, light-headedness, tunnel vision and eventually lead to a sudden loss of consciousness. The opposite case, where an excess level of blood oxygen is acquired (hyperoxia), leads to central nervous system (CNS) toxicity. Central nervous system toxicity is caused by short exposure to high partial pressures of oxygen at greater than atmospheric pressure. Pulmonary and ocular toxicity result from longer exposure to increased oxygen levels at normal pressure or normal concentrations at higher pressures. Symptoms may include disorientation, breathing problems, and vision changes such as myopia.

In the case of hyperoxia, deep-water divers are particularly at risk. As the diver descends in the water column, the partial pressure of each gas in the breathing mixture increases. As the partial pressure of oxygen increases beyond 1.4-1.6 atmosphere absolute (ATA), risk of an oxygen induced seizure increases and the shorter the exposure can be. Evidently, experiencing sudden seizures underwater may lead to drowning. Naturally, hyperoxia's potential impact on one's cognitive state may also impact other activities, such as for aircraft pilots or the like.

This background information is provided to reveal information believed by the applicant to be of possible relevance. No admission is necessarily intended, nor should be construed, that any of the preceding information constitutes prior art or forms part of the general common knowledge in the relevant art.

United States Patent Application Publication No. 2018/0001980 published January 4, 2018, to Hulbert and entitled "UNDERWATER SAFETY DEVICE" discloses a safety system for underwater diving which includes a biological sensor, an alarm system and a rescue deployment system. The safety system includes a controller which receives from the biological sensor sensed data. The controller determines whether sensed data is anomalous, that being either above or below a range of appropriate for the parameter by comparison to threshold values in a lookup table stored in memory. Different threshold values can be stored for different parameter types and/or different alert levels, and may be either default level(s) programmed in memory or user-definable threshold level(s). Determining whether a parameter value exceeds a threshold can include evaluating a rate of change of the parameter value, amongst other things.

Japanese Patent Application Publication No. 5124592 published May 21, 1993, to Osamu et al. has an English title of "DETECTOR OF CONSCIOUSNESS OF AIRCRAFT PILOT" and discloses a system for detecting the state of consciousness of a pilot based on monitoring brain oxygenation, as well as magnitude and rate of increase of acceleration. A near infrared light source and sensor are used to monitor brain oxygenation. The control device includes a storage device on which calibration data is stored and the amount of deoxidised hemoglobin is calculated by comparing the reflection intensity (or near-infrared light) with the calibration data. The storage device also stores data measured in advance, showing the relationship between the oxygen state in the pilot's brain, and magnitude and rate of increase of downward acceleration acting on the pilot.

### SUMMARY

A need exists for abnormal blood oxygenation level monitoring systems and methods (such a method is not covered by the scope of the claims), and self-monitoring oxygenation system and method (such a method is also not covered by the scope of the claims), that overcome some of the drawbacks of known techniques, or at least, provides a useful alternative thereto.

The invention is defined by a system according to claim 1. Further embodiments are defined by claims 2-5. No methods are covered by the scope of the claims.

### BRIEF DESCRIPTION OF THE FIGURES

Several embodiments of the present disclosure will be provided, by way of examples only, with reference to the appended drawings, wherein:
Figure 1 is a schematic diagram of a cerebral blood oxygenation monitoring system used by a scuba diver, in accordance with one embodiment.
Figure 2 is a diagram of a monitoring method for abnormal cerebral blood oxygenation levels of a user partaking in a physical activity requiring the use of an oxygen providing apparatus, the method not falling under the scope of the claims.
Figure 3 is an exemplary plot of the change in time of the relative absorbance of deoxyhemoglobin as measured by NIRS of an individual breathing a series of different gas mixtures containing lower than normal concentrations of oxygen (hypoxic mix), in accordance with one embodiment.
Figure 4 is an exemplary plot of the change in time of the relative absorbance of deoxyhemoglobin as measured by NIRS of an individual breathing a series of different gas mixtures containing higher than normal concentrations of oxygen (hyperoxic mix), in accordance with one embodiment.
Figure 5 is an exemplary plot of the change in time of the relative absorbance of both oxyhemoglobin and deoxyhemoglobin as measured by NIRS of an individual breathing normal air, an hyperoxic mix and normal air again, while changing position from a sitting position, a supine position and a sitting position again, in accordance with one embodiment;
Figure 6 is an exemplary plot of the change in time of the relative molar concentration of cerebral deoxyhemoglobin as measured by NIRS of an individual breathing different gas mixtures and immersed in water at different depths, in accordance with one embodiment;
Figure 7 is an exemplary plot of the relative change in concentration of cerebral deoxyhemoglobin as measured by NIRS of an individual breathing different gas mixtures inside a hyperbaric chamber, in accordance with one embodiment;
Figure 8 is an exemplary plot of the relative change in time of the concentration of cerebral deoxyhemoglobin as measured by NIRS of an individual both changing positions (sitting or supine) and breathing different gas mixtures inside a hyperbaric chamber, in accordance with one embodiment;
Figure 9 shows three exemplary plots illustrating the relative change over time in the concentration of cerebral deoxyhemoglobin; the heart rate and the breathing or respiration rate, from top to bottom respectively, of a user engaging in an underwater physical activity as a function of time, in accordance with one embodiment; and
Figure 10 shows a diagram of another method for monitoring a user's health risk for a user partaking in an activity requiring the use of an oxygen providing apparatus and/or inside a sealed pressurized environment, the method not falling under the scope of the claims.

Elements in the several figures are illustrated for simplicity and clarity and have not necessarily been drawn to scale. For example, the dimensions of some of the elements in the figures may be emphasized relative to other elements for facilitating understanding of the various presently disclosed embodiments. Also, common, but well-understood elements that are useful or necessary in commercially feasible embodiments are often not depicted in order to facilitate a less obstructed view of these various embodiments of the present disclosure.

### DETAILED DESCRIPTION

Various implementations and aspects of the specification will be described with reference to details discussed below. The following description and drawings are illustrative of the specification and are not to be construed as limiting the specification. Numerous specific details are described to provide a thorough understanding of various implementations of the present specification. However, in certain instances, well-known or conventional details are not described in order to provide a concise discussion of implementations of the present specification.

Various apparatuses will be described below to provide examples of implementations of the system disclosed herein. No implementation described below limits any claimed implementation and any claimed implementations may cover apparatuses that differ from those described below. The claimed implementations are not limited to apparatuses having all of the features of any one apparatus described below or to features common to multiple or all of the apparatuses described below. It is possible that an apparatus described below is not an implementation of any claimed subject matter.

Furthermore, numerous specific details are set forth in order to provide a thorough understanding of the implementations described herein. However, it will be understood by those skilled in the relevant arts that the implementations described herein may be practiced without these specific details. In other instances, well-known components have not been described in detail so as not to obscure the implementations described herein.

In this specification, elements may be described as "configured to" perform one or more functions or "configured for" such functions. In general, an element that is configured to perform or configured for performing a function is enabled to perform the function, or is suitable for performing the function, or is adapted to perform the function, or is operable to perform the function, or is otherwise capable of performing the function.

It is understood that for the purpose of this specification, language of "at least one of X, Y, and Z" and "one or more of X, Y and Z" may be construed as X only, Y only, Z only, or any combination of two or more items X, Y, and Z (e.g., XYZ, XY, YZ, ZZ, and the like). Similar logic may be applied for two or more items in any occurrence of "at least one ..." and "one or more..." language.

The systems described herein provide, in accordance with different embodiments, different examples in which a system for monitoring for abnormal blood oxygenation levels of a user, for example partaking in an activity while exposed to partial oxygen pressures deviating from the normal value of 0.21 at Standard Temperature and Pressure (STP). Namely, the systems, according to different embodiments, may be used to monitor blood oxygen content (bounded to hemoglobin and/or dissolved in the blood or tissues) and assess accordingly a health-related risk of hyperoxia and optionally hypoxia; or optionally derive therefrom assessment of the user's cognitive level. In the following examples, according to some embodiments, the user is usually constrained either to wear a breathing mask/apparatus or is located in a sealed and pressurized environment. This includes the user breathing gas that is either an hyperoxic or hypoxic mix (at any pressure) or normal air (e.g. 21% O₂) at a higher or lower pressure than atmospheric pressure. Examples of applications include, without limitation, underwater or deep diving, any activity in a hyperbaric chamber or deep-water bell or habitats (including hyperbaric medicine), any activity in a pressurized cockpit (e.g. piloting aircrafts, spacecrafts) and EVA suits or similar. Other users may include monitoring oxygenation during first-aid (including using defibrillators), for firefighters, soldiers, etc.

In some embodiments, the systems described below rely on various oximetry techniques (e.g. pulse or cerebral oximetry, etc.) to identify and quantify the presence of one or more chromophores' molecules in the user's blood. The measured attenuation (or optical density) measured from one or more oximetry probes may be used to derive a corresponding oxygen partial pressure and/or relative oxygen concentration in said blood. This includes, in some embodiments, quantifying the concentration of dissolved O2 (dO2) by monitoring the oxygen input, levels of mixed blood saturation, combined with a physiological model of oxygen transport in the body. Thus, the systems described herein, in accordance with some embodiments, may be used to monitor in real-time a user's health risk of hyperoxia and optionally hypoxia in such operating environments.

The oximetry technique used is based on near-infrared spectroscopy (NIRS). These are based on the fact that distinct biological molecules change their optical properties when binding to oxygen. This phenomenon is caused by the fact that chromophores such as oxygenated hemoglobin (oxyhemoglobin or O2Hb) differs in parts of its absorption pattern from de-oxygenated hemoglobin (deoxyhemoglobin or HHb), and thus in their apparent optical spectrum. These optical differences have been exploited and are now clinical standard application in pulse oximetry, where usually two or three distinct wavelengths are used in combination with pulse plethysmography to measure the arterial hemoglobin oxygen saturation. Visible light penetrates tissue only short distances, since it is markedly attenuated by several tissue components, which absorb or scatter visible light. However, in the near-infrared (NIR) spectrum (ranging from 700 to 1100 nm) photons are capable of deeper penetration of several centimeters or more. Moreover, NIR beams may also penetrate bones, which is prerequisite for trans-cranial cerebral oximetry for example, although generally speaking other probe locations may be used. Aside from the advantage of relatively deep penetration of several centimeters, the NIR spectral region is also characterized by typical differences in the spectrum of oxygenated and deoxygenated hemoglobin, for example. As mentioned above, other chromophores present in the blood that may be monitored using these techniques usually comprise O2Hb and HHb, but other molecules may be monitored as well, for example (and without limitation) cytochrome c oxidase, carbon monoxide (CO), methemoglobin, etc.

In some embodiments, it may also be that one or more chromophores being monitored have more complex absorption spectra, such as a broader spectrum and/or comprising of two or more peaks. Generally speaking, the herein described embodiments are not limited to using one to three wavelengths, but may use as many wavelengths as needed to properly characterize the presence of one or more chromophore molecules present in the user's blood. To achieve this, any number of additional wavelengths may be used (e.g. any wavelength ranging from 600 to 1100 nm). Furthermore, measuring such components may result in overlapping spectra features for two or more components. In this case, multivariate statistical analysis methods may be applied to extract a singular signature for each overlapping component. For example, these may include, without limitation: linear (or non-linear) multivariate regression (MVR), principal component analysis (PCA), principal component regression (PCR), discriminant analysis (DA), hierarchical cluster analysis (HCA), soft independent modeling of class analogy (SIMCA), or similar.

Exploiting these natural characteristics for regional oximetry such as cerebral oximetry (or other), a prototypical NIRS probe functions as follows: A light source (e.g., one or more LEDs of different wavelengths) generates NIR light, concerning the spectrum centered around characteristic wavelengths. The emitted beam is directed into the tissue of interest via a (usually cutaneous attached) probe. The probe is usually attached to the skin above the tissue of interest. Respective stickers of the probes serve to stabilize the probe's position over longer periods, but also restrict entrance of ambient light into the measurement photon pathway. Transcutaneous NIRS is noninvasive and the applied light intensities are not harmful to the tissue, not causing skin burns even if applied for a longer period.

Generally, the change in molar concentration of the monitored chromophore (for example O2Hb or HHb) may be calculated from the measured change absorbance/attenuation of the NIRS signal by using a physical model of light diffusion and attenuation in organic tissues derived from radiative transfer theory (e.g. using a modified Beers-Lambert law or similar; for example see: Susumu Suzuki, Sumio Takasaki, Takeo Ozaki, and Yukio Kobayashi "Tissue oxygenation monitor using NIR spatially resolved spectroscopy", Proc. SPIE 3597, Optical Tomography and Spectroscopy of Tissue III, (15 July 1999); doi: 10.1117/12.356862 and Michael S. Patterson, B. Chance, and B. C. Wilson, "Time resolved reflectance and transmittance for the non-invasive measurement of tissue optical properties," Appl. Opt. 28, 2331-2336 (1989).

Furthermore, in some embodiments, the molar concentration may then be used to assess, for example, the dissolved oxygen content in the user's blood (dO2). Indeed, oxygen is found in two forms in the blood: in solution (or dissolved) and bound to hemoglobin. Since dissolved oxygen may accumulate in the blood and be discharged at a later time when partial oxygen pressures are lowered, such an assessment may be important for assessing a user's risk level. In some embodiments, a physiological model may be used to calculate the concentration of dO2 (or change thereof). For example, such a model may determine, in some embodiments, the component or fraction of the inhaled oxygen that is absorbed into the blood stream from measurement of the partial pressure of the inhaled gas mix since the inhaled and absorbed gases will reach equilibrium across the alveolar-blood interface. Current knowledge of physiological processes (such as Fick's diffusion law, O2 solubility, etc.) allows for this evaluation. For example, assuming that the oxygen that enters the blood stream is either bounded to hemoglobin or remains in a dissolved state, changes in the oxy and deoxyhemoglobin concentrations in the target mixed blood volume can be evaluated. This in turn allows a determination of the component that remains in the dissolved state under the assumption that other physiological parameters such as total hemoglobin number, blood volume, etc., remains nominal and constant. In some embodiments, such a model may use additional parameters such as the oxygen intake (e.g. quantity of oxygen inhaled) for example derived from a measurement of the flow rate of the inhaled gas mix, ambient pressure, an estimation or measurement of the user's blood volume, etc. In some embodiments, an index of user dO2 levels may be constructed for reference.

Other user body parameters may be used, for example and without limitation, parameters related to the user's weight/height, age and/or physical fitness.

In some embodiments, the monitoring systems described herein may further be used to derive a cognition level or index of the user at different levels of blood oxygenation (bounded to hemoglobin and/or dissolved in blood or tissues). The cognition level may include characterizations of user fatigue, stress, confusion, engagement, workload and may be used to assess the ability of the user to concentrate and/or accomplish different tasks (e.g. efficiency and precision), such as diving, piloting an aircraft or spacecraft, etc. The systems and methods described herein, in some embodiments, may further display the user's cognition level in addition to health-related risks of hyperoxia and/or hypoxia. The cognition level may be derived, for example, by initially assessing the user's ability to execute specific tasks (i.e. speed of execution, number of errors, etc.) while monitoring changes in blood oxygen levels and deriving correlations from those measurements. In the case where the user's cognitive level is determined to be below a certain safety threshold for performing a specific task (i.e. flying an aircraft, etc.), the user may decide or be forced to stop and/or take a break.

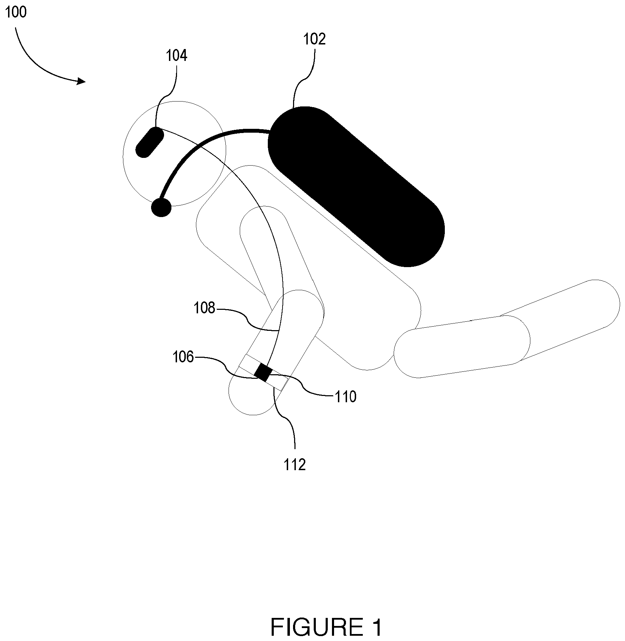

With reference to Figure 1, and in accordance with one exemplary embodiment, a cerebral blood oxygen monitoring system, generally referred to using the numeral 100, is shown. In Figure 1, the illustrated physical activity is scuba diving or deep diving. As noted above, while scuba diving or deep diving is provided herein as an example, other activities may be considered to benefit from the features, functions and advantages of the herein-described embodiments without departing from the general scope and nature of the present disclosure.

In the illustrated embodiment, the system 100 is configured to monitor for abnormal cerebral blood oxygenation levels of the diver underwater. In this exemplary embodiment, the diver uses a closed or semi-closed circuit rebreather device 102, though other oxygen-providing devices or means may be considered, for example, where a recycling of exhaled gases is not applied. As mentioned above, using such devices at great depths can lead to an increased partial oxygen pressure which itself may result in the onset of hyperoxia.

The system 100 comprises at least one near-infrared spectroscopy (NIRS) probe 104 fixable to the user's head for acquiring cerebral blood oxygenation data representative of at least deoxyhemoglobin levels over time. As mentioned above, other embodiments may be configured to monitor different/additional chromophore molecules present in the blood, such as oxygenated haemoglobin levels, without limitation..

In some embodiments, this at least one NIRS probe 104 may be integrated inside a type of headwear, such as a headband or cap. In this case, the headwear should be solidly affixed on the head of the user to avoid suboptimal measurements due to a suboptimal contact between the NIRS probes and the user's skin, water contamination or the like. As will be discussed below, other embodiments may use different skin contact locations, for example and without limitation, the neck region.

From the absorption spectra measured from this at least one NIRS probe, a relative cerebral (or regional) blood levels of these proteins may be calculated. To do so, the at least one NIRS probe 104 is operatively connected to a digital data processor 106 programmed to compute the relative concentrations of both O2Hb and HHb, and/or a change in molar concentration of HHb or similar. In this embodiment, data is transferred through a wired connection 108, but other embodiments, such as wireless connections, may also be employed. As will be explained in more detail below, the digital data processor 106 is further programmed to use these relative concentration measurements to derive or define at least a lower or higher health risk rating of hyperoxia, and/or other oxygenation health-related ratings, such as related to hypoxia, for example.

It will be appreciated that the processor 106 may take various forms, which may include, but is not limited to, a dedicated computing or digital processing device, microprocessor, a general computing device, tablet and/or smartphone interface/application, and/or other computing device as may be readily appreciated by the skilled artisan, that includes a digital interface to a at least one NIRS probe output so to acquire and ultimately process readings/spectra captured thereby.

Furthermore, the embodiment of Figure 1 further comprises a digital user interface 110 capable of displaying a health risk indicator to the user obtained via the digital data processor 106. As shown in the embodiment of Figure 1, both the digital data processor 106 and the digital user interface may be contained inside the same water-tight device, here a watch-like device worn on the wrist using a strap 112. In other embodiments, the digital user interface 110 and digital data processor 106 may also be separated from each and communicatively linked to each other and to the at least one NIRS probe via a wired or wireless connection. In some embodiments, the digital user interface may be comprised of a computer with a digital display screen, tablet, smartphone application or like general computing device, or again a dedicated device having a graphical or like general computing device. In some embodiments, the digital user interface may comprise a heads-up display located inside a mask, goggles and/or glasses (not shown).

In some embodiments, additional sensors may also be used in parallel with the at least one NIRS probe 104. For example, pressure, temperature sensors (and/or one or more same and/or distinct physiological sensors or like components operable to interface with the user (e.g. via a direct or indirect user contact, such as a skin contact or like interface operable in contact with or in close proximity to the user's skin or body) may also be used to acquire environmental and/or physiological signals and operatively connected to the digital data processor 106, either for direct transmission to the digital user interface 110 or to be used as additional input in the determination of the user's higher or lower health risk rating of hyperoxia, hypoxia, etc. Examples of physiological signals that may be monitored via one or more physiological sensor include, without limitation, electrocardiograms (ECG), electroencephalograms (EEG), breathing rate, VO2, blood pressure, body temperature, etc. As will be discussed below, in some embodiments, one or more physiological signal may be correlated with the NIRS probe signal to provide a more precise quantification of blood oxygen levels.

In some embodiments, user body position may also be monitored with one or more accelerometers (not shown), as the user body position affects the flow of blood to the head region (as will be explained below) and thus the spectral response. Therefore, in some embodiments, system 100 may further comprise one or more accelerometers communicatively linked to digital data processor 106 to detect changes in user body position or orientation (e.g. sitting or supine).

In some embodiments, system 100 may further comprise an internal memory or data storage module (not shown) communicatively linked to digital data processor 106 to store additional data which may be used to improve the monitoring capabilities of system 100. For example, and without limitation, a spectral database comprising information about the spectral signature of one or more known chromophores may be stored therein.

In some embodiments, digital data processor 106 may further be configured to provide additional features, such as an artificial-intelligence-based monitoring system (not shown). In some embodiments, digital data processor may be configured to run an artificial intelligence program to provide user-specific automated or semiautomated oxygen monitoring, as will be explained below.

Furthermore, digital data processor 106 may also, in some embodiments, be communicatively linked to the oxygen providing apparatus/device 102 so as to regulate the flow of gas to the user, depending on the user's blood oxygen levels being monitored.

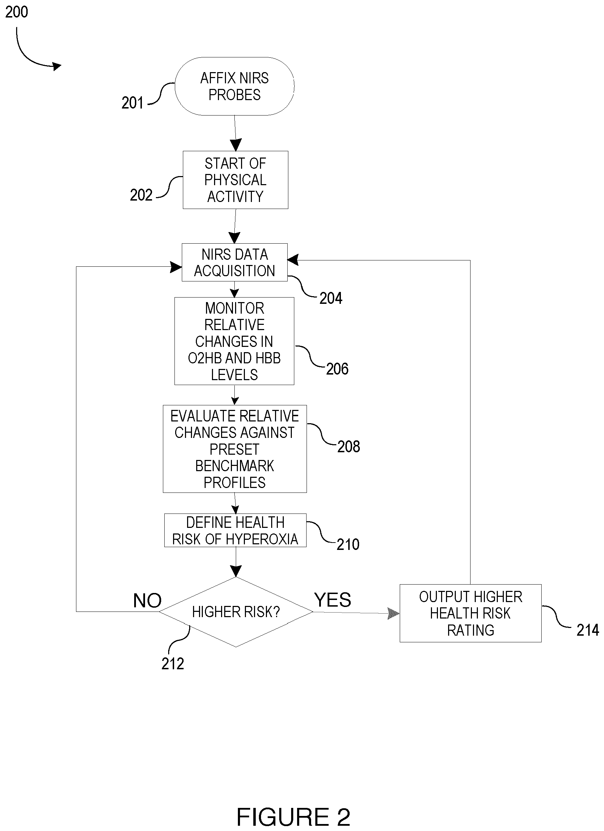

With reference to Figure 2 and in accordance with one example not forming part of th claims, a method for deriving a health indicator from measurements taken using the at least one NIRS probe will now be described for a user partaking in an activity requiring the use of an oxygen providing apparatus. The user first affixes (step 201) at least one near-infrared spectroscopy (NIRS) probe to, for example, his/her head. As explained above, the at least one probe may be integrated within a form of headwear, although generally any wearable user body location may be used. Non-limiting examples of suitable wearable monitoring device comprising one or more probes may include, but are not limited to, a wristband, wristwatch, bracelet, necklace, ring, belt, glasses, clothing, hat, anklet, headband, chest harness, patch, skin probe to name a few, or any other wearable item location that is capable of obtaining a NIRS signal.

The user then starts partaking in any activity (such as a physical activity or other) as usual (step 202) while the at least one probe acquires data relative to his/her oxyhemoglobin (O2Hb), deoxyhemoglobin levels (HHb) or other chromophore levels as mentioned above (step 204). This data acquisition is done continuously, in real-time or at short intervals. In the presently discussed embodiment, the acquired data is analyzed by monitoring for relative changes in O2Hb and HHb levels (step 206). These relative changes are automatically evaluated against present variations corresponding to a plurality of benchmark cerebral blood oxygenation profiles (step 208). These profiles are determined beforehand and programmed, for example, into the digital data processor 106 as explained above. As mentioned above, the profile may further comprise, in some embodiments, data related to one or more physiological signals, which would be acquired concurrently using one or more physiological sensors. Furthermore, as discussed before, The profile themselves are associated with a preset blood oxygenation index that defines at least a lower health risk rating and a higher health risk rating of hyperoxia (step 210) and/or other oxygenation health-related characteristics.

As mentioned above, method 200 may also use at step 210 an artificial-intelligence-based system to provide an improved monitoring capabilities of the user's oxygen levels and related risks of hyperoxia/hypoxia. Such a system may receive and analyze in real-time any data being acquired via the NIRS probe, one or more physiological sensors, user-body parameters, total oxygen intake, manual changes in the oxygen content flow rates, etc. Different AI, machine learning and/or system automation techniques may be considered to implement such a program. For example, these may include, without limitation, supervised and/or unsupervised machine learning techniques, linear and/or non-linear regression, decision trees, etc. Deep learning algorithms may also be used, including but not limited to, neural networks such as recurrent neural networks, recursive neural networks, feed-forward neural networks, convolutional neural networks, deep-belief networks, multi-layer perceptrons, selforganizing maps, deep Boltzmann machines, and stacked de-noising auto-encoders or similar. As such, the intelligent monitoring features may operate autonomously or semiautonomously, with limited or without explicit user intervention.

Using hyperoxia as an example, if the method determines at step 212 that the user is experiencing a lower risk of hyperoxia, nothing is done and the method continues the process of acquiring data of step 204. In contrast, if the method determines that the user is currently experiencing a higher health risk of hyperoxia, the method then outputs the higher health risk rating to the user (step 214) to inform him/her of the higher risk so that he/she may take action to reduce it. Different examples of indicators may include, but are not limited to, visible indicators such as flashing and/or coloured lights, audible alerts (e.g. relayed through a communicatively-linked earpiece), vibratory device, or the like, which may take the form of continuous, blinking, pulsing, rhythmic, periodic and/or escalating alerts indicators. In some embodiments, visual indicators may be shown on a digital display or a heads-up display, as mentioned above.

Then, as in the previous case, the method continues the process of acquiring data (step 204). The paragraphs below will explain, in part, how the cerebral blood oxygenation profiles may be determined.

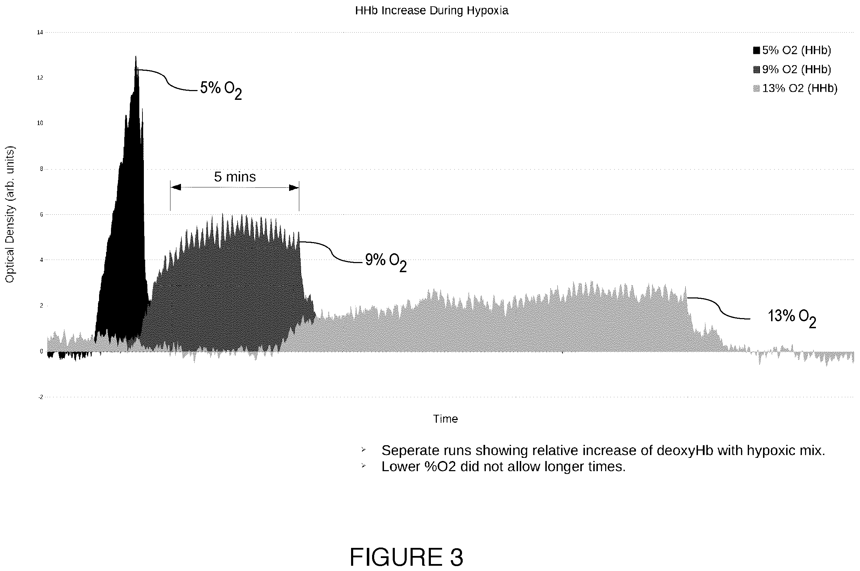

With reference to Figure 3, and in accordance with one exemplary embodiment, a plot is provided of the relative change in cerebral deoxyhemoglobin (HHb) absorbance (e.g. optical density), as a function of time, of an individual breathing a series of gas mixtures with a reduced oxygen concentration (hypoxic mixes). The measurements were taken using a commercially available NIRS system developed by Artinis Medical Systems B.V. The absorbance values are relative to the baseline values obtained with the same individual breathing normal air (21% O₂) and three runs were measured with hypoxic mixes of 5%, 9% and 13% oxygen respectively. For each data series, the individual sustained breathing the associated mix as long as comfortable, then returned to breathing normal air again. Clearly, breathing lower levels of oxygen, as is well known, leads to a rapid increase in HHb levels. The lower the oxygen level, the faster and higher the rise in measured HHb levels is observed and the shorter the time the individual could sustain respiration.

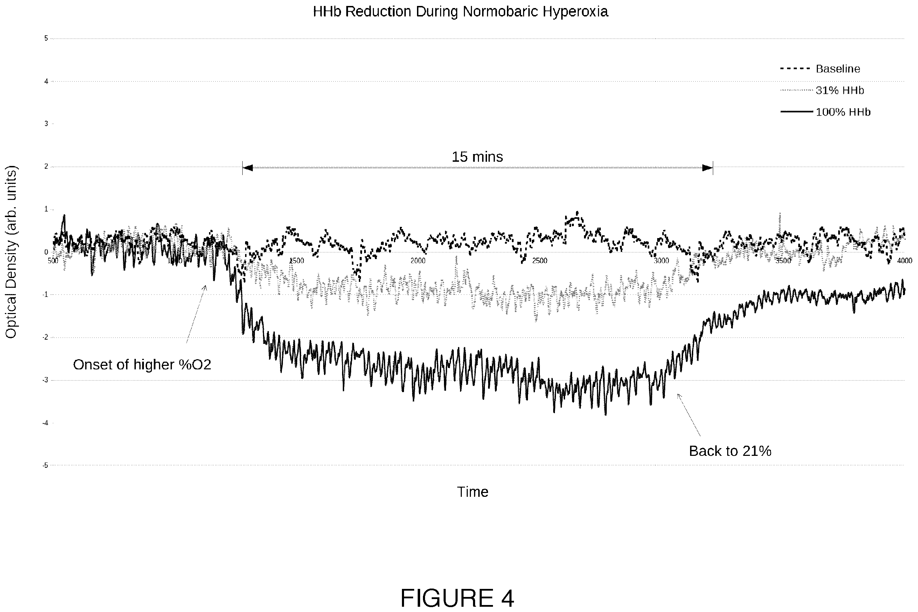

In contrast to Figure 3, Figure 4 is a plot, as a function of time, of the change of HHb levels while breathing an increased concentration of O₂ (hyperoxic mix). Three measurements are shown, one baseline measurement at a normal O₂ concentration of 21% (e.g. normal air) (dark gray dotted line), one measurement done with a mix containing 31% O₂ (light gray dotted line) and one measurement with pure O₂ (black line). In the last two measurement series, the individual was breathing normal air in the first and last 5 minutes of the experiment. We clearly see the reduction in HHb concentration measured with the increased intake in O₂. Moreover, while the measurements at 31% O₂ show a quick return to the baseline value after the individual stopped breathing the gas mixture, in the second case, while the HHb concentration increases and stabilizes after the pure O₂ is removed, it never quite returns to baseline during the acquisition time, though will clearly eventually return to baseline over time. These measures thus illustrate the tissue's ability to store oxygen, which may become increasingly important for greater oxygen partial pressures. Using methods as described herein, in some embodiments, means may thus be provided to monitor the discharge of oxygen from tissues into the blood.

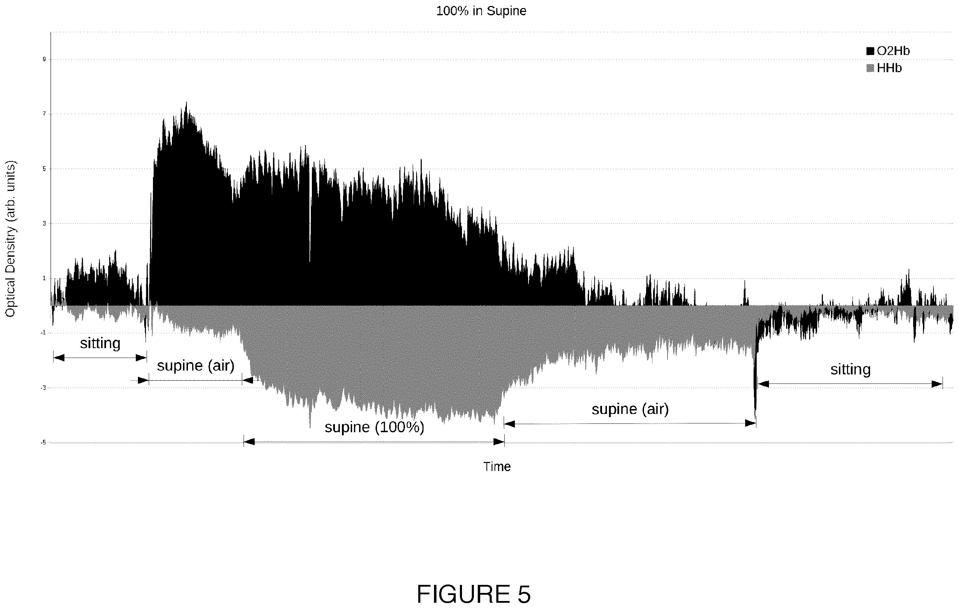

Figure 5 shows the effect, as a function of time, of both changing an individual's position (sitting or supine) and breathing pure oxygen (100% O₂) vs. normal air (21% O₂). Both the relative absorbance values of the oxyhemoglobin (O2Hb) and HHb are shown. For this experiment, the individual is initially breathing normal air (21% O₂) in a sitting position for 5 minutes, followed by being put in a supine position for another 5 minutes. The individual, still in a supine position, was then exposed to a pure oxygen gas via a face mask for a number of minutes. Without changing the individual's position, the mask was then removed, allowing the individual to breath normal air again. Finally, after waiting a few minutes, the individual was allowed to sit again. We clearly see the effects these changes have on both the O2Hb and HHb measurements. However, we find that the O2Hb and HHb responses are not symmetrical, indicating that measurement only the O2Hb concentration may be unreliable as the only indicator of cerebral blood oxygenation in all contexts. However, a careful measurement of both O2Hb and HHb concentrations using NIRS does lead to the determination of a more precise index.

Figures 3 to 5 clearly show characteristic signatures of the changes in O2Hb and HHb levels not only as a function of oxygen content breathed by an individual but also as a function of the individual's relative position. By measuring the changes in O2Hb and HHb levels for a series of different oxygen levels in different individuals, a series of benchmark cerebral blood oxygenation profiles may be recorded. These profiles may then be digitally associated with a preset cerebral blood oxygenation index that may then be used to associate a lower or higher risk rating of hyperoxia in the individual, of example. The benchmark profiles may be expanded to include different partial oxygen pressures by doing measurements inside a hyperbaric or isobaric chamber, for example.

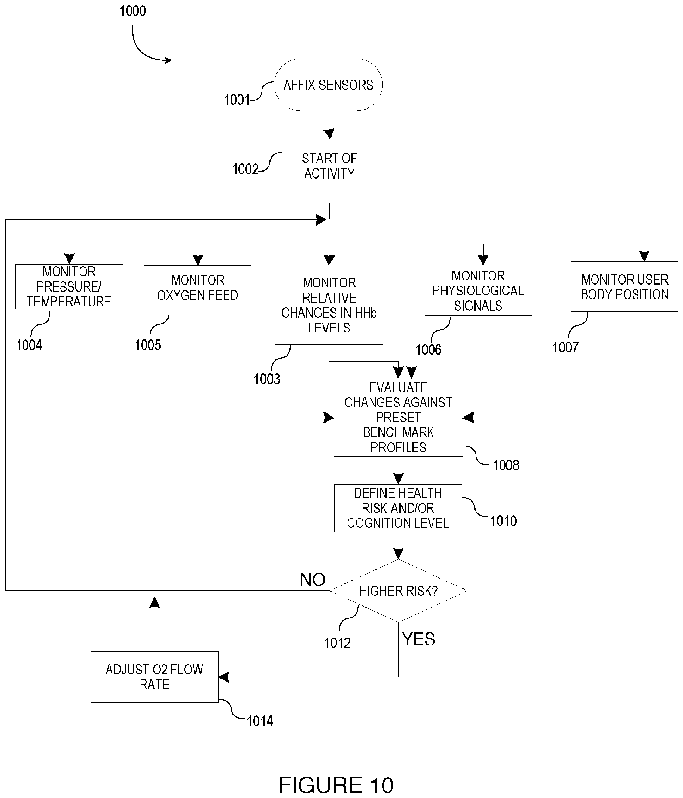

With reference to Figure 10 and in accordance with one exemplary embodiment, another method for monitoring a user's health risk when partaking in an activity requiring the use of an oxygen providing apparatus and/or inside a sealed pressurized environment, generally referred to using the numeral 1000, will now be described. Similar to the embodiment described in Figure 2, at step 1001 one or more sensors are affixed or put in contact with the user's skin at one or more locations. These sensors may be integrated into a wearable device as explained above. Once the user begins the activity at step 1002, method 1000 immediately starts monitoring one or more parameters. At step 1003, the method monitors via one or more NIRS probes the molar concentration of HHb in the user's blood (for example in the cerebral region), but may also optionally monitor in parallel other parameters such as ambient pressure and/or temperature (step 1004), oxygen intake (step 1005), one or more physiological signals via one or more physiological sensors (step 1006) and/or user body position via one or more accelerometers (step 1007). Data acquired from steps 1003 to 1007 is sent to a central processing unit (i.e. digital processing unit 106 for example) to be analyzed and compared to preset benchmark profiles at step 1008. As discussed above, in some embodiments, step 1008 may be performed using machine-learning techniques such as deep learning techniques or similar. From this analysis, a health-related risk of hyperoxia/hypoxia and optionally a user cognition level may be defined at step 1010. At step 1012, these risk and/or cognition levels may be compared to previous levels to determine if an increase in risk or a loss of cognition has occurred. In this case, method 1000 may automatically adjust the flow of oxygen delivered to the user to reduce the risk and/or increase the cognition level. Optionally, at step 1014 a warning may also be delivered to the user as explained above. The method then goes back to monitoring different parameters (steps 1003 to step 1007) to assess a new risk and/or cognition level.

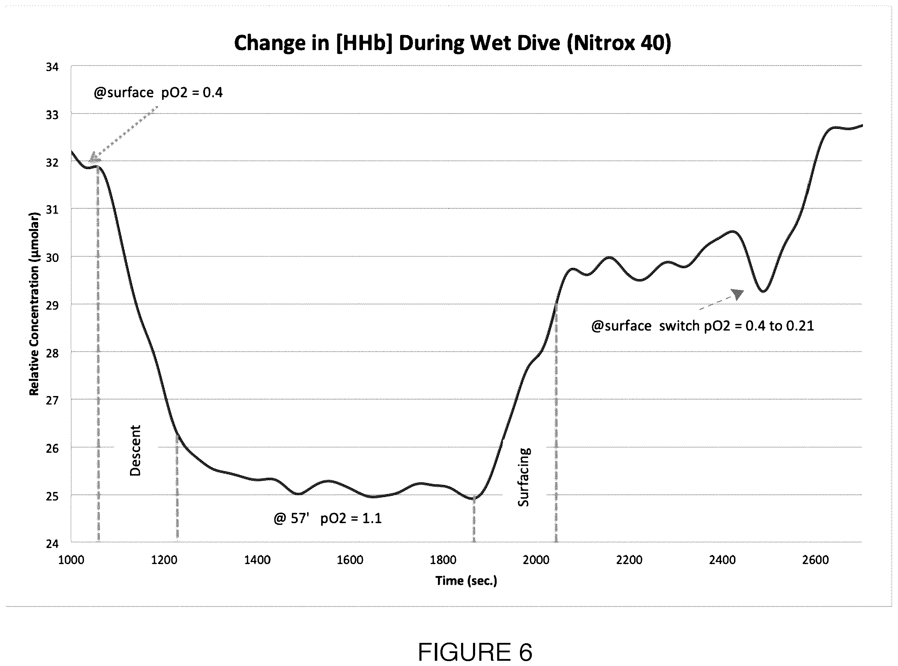

With reference to Figures 6 to 9, and in accordance with one exemplary embodiment, different plots are provided of the change in cerebral HHb concentration (in µM or 10⁻⁶ mol/L), as a function of time, of an individual subjected to different oxygen partial pressures. These figures clearly show the different correlations between the measured molar concentration of HHg different parameters, including partial oxygen pressure but also sustained physical activity. The measurements were again taken using a commercially available NIRS system developed by Artinis Medical Systems B. V. Changes in molar cerebral HHb concentrations were calculated from changes the NIRS attenuation signal using the light diffusion model of Suzuki et al. mentioned above. Thus, in Figures 6 to 9, only changes in the measured cerebral HHg concentration with respect to the initial value are meaningful and the initial concentration value at the start of each Figure is arbitrary.

For example, Figure 6 shows a plot of an individual being completely immersed in water at different depths and breathing sequentially from two different gas mixtures (normal air and Nitrox 40 hyperoxic mix). At the start of the plot shown in Figure 6, the individual is breathing the Nitrox 40 mix while floating at the surface (e.g. p02 = 0.4). As the depth increases, we clearly see the concentration of cerebral HHb decreasing as well with respect to the initial value (at the surface at t = 2000 sec.) by about 7 µM until a depth of 57 feet is reached with a corresponding partial oxygen pressure of 1.1. The diver stayed at that depth for about 10 minutes before resurfacing at around 2400 sec., where we see the concentration of HHb increasing correspondingly by about 2.5 µM, returning it close to its initial value at the start of the experiment. Thus Figure 6 shows a clear correlation between changes in oxygen partial pressure and corresponding changes in cerebral HHb concentration.

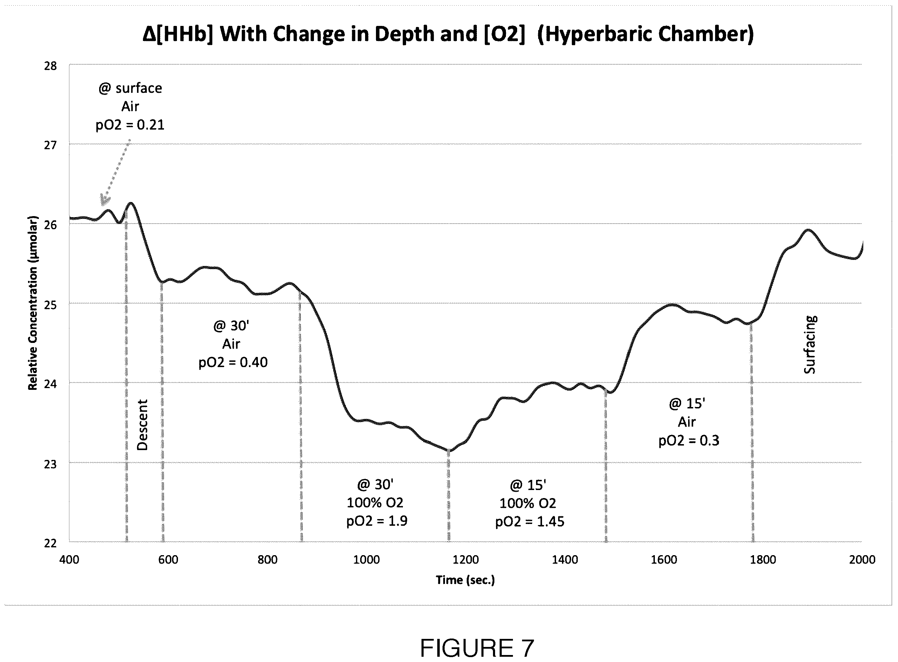

Similarly, Figure 7 shows a plot of the change in cerebral HHb concentration as a function of time but for an individual inside a sealed hyperbaric chamber where both a change of O2 concentration was administered and a change in depth simulated by varying the pressure. Thus, the partial oxygen pressure inside the user could be changed by either changing the pressure in the chamber or by changing the oxygen concentration the individual was breathing (air or hyperoxic mix). Starting from a normal oxygen partial pressure of 0.21 (e.g. breathing normal air at atmospheric pressure), the pressure inside the chamber was increased to simulate a corresponding depth of 30 feet (pO2 = 0.40) which led to a small decrease in cerebral HHb concentration (with respect to its initial value at t = 400 sec.) of about 0.8 µM. Then, at around 900 seconds, the pressure was kept constant but the breathing mix was changed from air to pure oxygen (pO2 = 1.9). We quickly see the HHb concentration further decreasing by a value of about 2 µM. The next step consisted of letting the individual breathe pure oxygen but to decrease the pressure to simulate a depth of 15 feet (pO2 = 1.45). We see that this leads to a corresponding increase of the HHb concentration by about 0.8 µM. Then, keeping the pressure constant, the individual was given normal air to breathe (p.02 = 0.3). We again find a corresponding rapid increase in the concentration of cerebral HHb by a value of 1 µM. Finally, the normal atmospheric pressure was restored which led the concentration to return to its initial value (at t = 0). Thus, we clearly see in Figure 7 the correlation between changes in depth and partial oxygen pressure and the corresponding variations in the cerebral HHb concentrations.

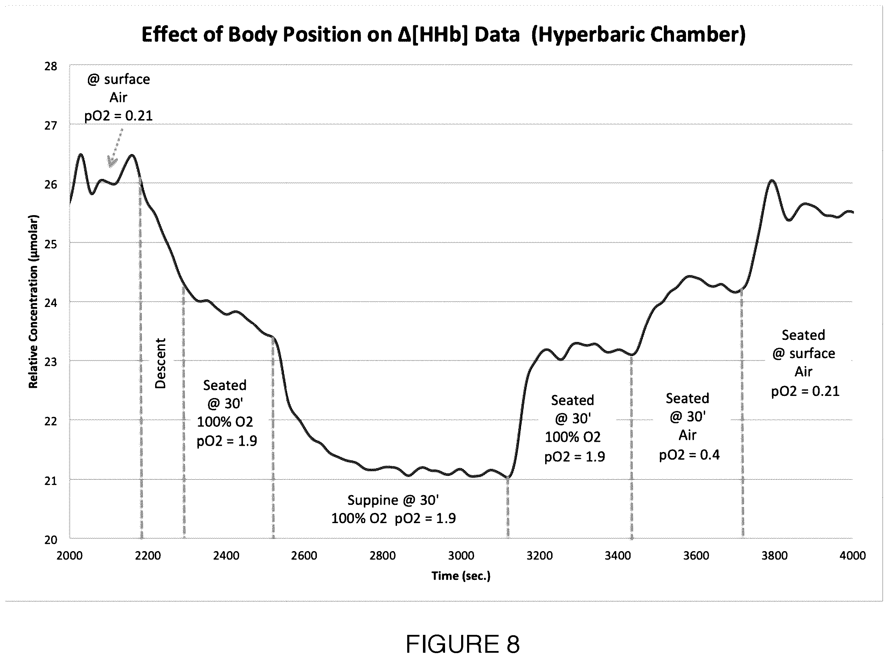

Figure 8 illustrates, similarly to Figure 5, the effect, as a function of time, of both changing an individual's position (sitting or supine) and breathing pure oxygen (100% O2) vs. normal air (21% O2), again inside a hyperbaric chamber. In the plot of Figure 8, the individual is first in a seated position while breathing normal air. The pressure was then increased to a corresponding depth of 30 feet, resulting in a corresponding decrease in the HHg concentration by about 2 µM with respect to its initial value (t = 2000 sec.). Then, the individual, still seated, was administered pure oxygen (pO2 = 1.9) which leads to another decrease of the HHg concentration by about 0.6 µM. Next, keeping the pressure constant (30 feet) and still breathing pure oxygen, the individual was asked to take to a supine position, which causes the measured concentration of cerebral HHb to decrease further by about 2 µM. Going back to a seated position cancelled, as expected, the previous variation by increasing the HHg concentration by 2 µM. Now changing the breathing mix from pure oxygen to normal air (but keeping the pressure constant and a seated position) also returned the cerebral HHg concentration to a value of about 1.9 µM below the initial value. Finally, decreasing the pressure to normal atmospheric pressure returned the measured HHg concentration close to the initial value at the start of the experiment. Thus, we see that the derived molar concentration also correlates well with the user body position.

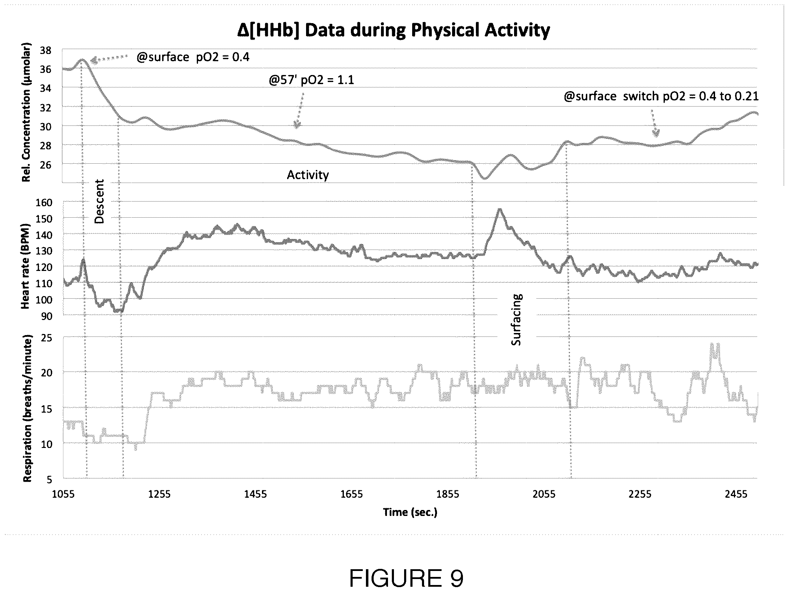

As mentioned above, in some embodiments, one or more physiological signals may be acquired concurrently with the NIRS signal to provide an increased accuracy in the calculated blood oxygen content, for example by using measured correlations between the changes in these one or more physiological signals and the HHb concentration levels (or other chromophores) when the user is engaging in a physical activity. For example, in Figure 9 we see three plots illustrating the corresponding change over time of the concentration of cerebral HHb, the user's heart rate (in beat per minute or BPM) and the breathing or respiration rate (in breaths per minute), from top to bottom respectively, of a user engaging in an underwater physical activity as a function of time. In Figure 9, the individual is initially breathing an hyperoxic mix (pO2 = 0.4) while at rest at the surface and then descends underwater to a depth of 57 feet (pO2 = 1.1), leading to a corresponding decrease in the measured cerebral HHb concentration of about 5 µM below the initial value (t = 1055 sec.). This decrease is also correlated with a small decrease in the heart rate from 120 BPM to about 95 BPM. The individual then started engaging in a physical activity for more than 10 minutes, which immediately results in an increase in the measured heart rate (from 95 BPM to about 128 BPM with peak at 140) and the respiration rate (from about 10 breaths per minutes to around 19-20 breaths per minute), and a corresponding decrease of the HHb concentration by about 4 µM (e.g. 10 µM below the initial value). This decrease in the HHg concentration is directly linked to the physiological processes caused by the physical activity being performed and not linked to the partial oxygen pressure alone, as will be seen below. Following this, the individual returns to the surface while still breathing the hyperoxic mix, which shows as a slight increase in the HHb concentration by a value of about 4 µM. Finally, the diver resumes breathing normal air, which shows up again as an increase in the HHb concentration of about 4 µM. Thus, the measured cerebral HHg concentration at the end is still 4 µM below the initial value at the start of the experiment, which roughly corresponds to the decrease observed when the user was engaged in the physical activity, as expected.

## Claims

1. A system (100) for monitoring for hyperoxia for a user, the system (100) comprising:
an optical spectroscopy probe (104) fixable to the user's skin for acquiring blood oxygenation data representative of deoxyhemoglobin levels over time, wherein said optical spectroscopy probe (104) comprises a near infrared spectroscopy probe, and wherein the user is exposed to partial oxygen pressures deviating from a standard value of about 0.21 atm at Standard Temperature and Pressure or is exposed to a pressure other than 1 atm;
a digital user interface (110) operable to display a health risk indicator to the user; and
a digital data processor (106) operatively connected to said optical spectroscopy probe (104) and user interface (110), and programmed to:
monitor variations in said deoxyhemoglobin levels;
automatically evaluate said variations in said deoxyhemoglobin levels against respective preset deoxyhemoglobin level variations forming part of benchmark blood oxygenation profiles, wherein said profiles are digitally associated with a preset blood oxygenation index defining at least a lower health risk rating and a higher health risk rating of hyperoxia; and
output a signal representative of said higher health risk rating of hyperoxia in response to said evaluation for display via said digital user interface (110).

2. The system of claim 1, for monitoring a user exposed to pressures above 1 atm.

3. The system of any one of claims 1 to 2, for monitoring a user partaking in an activity requiring use of an oxygen providing apparatus (102).

4. The system of any one of claims 1 to 3, for monitoring a user partaking in an activity requiring use of an oxygen providing apparatus (102), wherein the oxygen providing apparatus comprises a rebreather, and wherein said digital data processor (106) is operatively linked to a rebreather controller to automatically adjust a rebreather output, in the form of gas flow to the user, in response to said higher health risk rating.

5. The system of any one of claims 1 to 4, wherein said blood oxygenation data is further representative of oxygenated hemoglobin levels, wherein said digital data processor (106) is further programmed to monitor and evaluate relative variations in said oxygenated hemoglobin levels.

## Patentansprüche

1. Ein System (100) zur Überwachung auf Hyperoxie für einen Benutzer, wobei das System (100) Folgendes umfasst:
eine optische Spektroskopiesonde (104), die an der Haut des Benutzers befestigt werden kann, um Blutsauerstoffsättigungsdaten zu erfassen, die den Desoxyhämoglobinspiegel im Zeitverlauf darstellen, wobei die optische Spektroskopiesonde (104) eine Nahinfrarot-Spektroskopiesonde umfasst und wobei der Benutzer Sauerstoffpartialdrücken ausgesetzt ist, die von einem Standardwert von etwa 0,21 atm bei Standardtemperatur und -druck abweichen, oder einem anderen Druck als 1 atm ausgesetzt ist;
eine digitale Benutzeroberfläche (110), die so betrieben werden kann, dass sie dem Benutzer einen Gesundheitsrisikoindikator anzeigt, und
einen digitalen Datenprozessor (106), der operativ mit der optischen Spektroskopiesonde (104) und der Benutzeroberfläche (110) verbunden und so programmiert ist, dass er Folgendes ausführt:
Überwachung von Schwankungen des Desoxyhämoglobinspiegels;
automatische Auswertung von Schwankungen des Desoxyhämoglobinspiegels bezogen auf entsprechende voreingestellte Schwankungen des Desoxyhämoglobinspiegels, die Teil von Benchmark-Blutsauerstoffsättigungs-Profilen sind, wobei die Profile digital mit einem voreingestellten Blutsauerstoffsättigungs-Index verknüpft sind, der mindestens eine niedrigere Gesundheitsrisikoeinstufung und eine höhere Gesundheitsrisikoeinstufung bezüglich Hyperoxie definiert, und
Ausgabe eines Signals als Reaktion auf die Bewertung, das für die höhere Gesundheitsrisikoeinstufung bezüglich Hyperoxie repräsentativ ist, zur Anzeige über die digitale Benutzeroberfläche (110).

2. Das System nach Anspruch 1 zur Überwachung eines Benutzers, der Drücken über 1 atm ausgesetzt ist.

3. Das System nach einem der Ansprüche 1 bis 2 zur Überwachung eines Benutzers, der an einer Aktivität teilnimmt, die die Verwendung eines Sauerstoffbereitstellungsgeräts (102) erfordert.

4. Das System nach einem der Ansprüche 1 bis 3 zur Überwachung eines Benutzers, der an einer Aktivität teilnimmt, die die Verwendung eines Sauerstoffbereitstellungsgeräts (102) erfordert, wobei das Sauerstoffbereitstellungsgerät ein Kreislaufatemgerät umfasst und wobei der digitale Datenprozessor (106) operativ mit einer Kreislaufatemgerät-Steuereinheit verbunden ist, um als Reaktion auf die höhere Gesundheitsrisikoeinstufung automatisch die Ausgabe des Kreislaufatemgeräts in Form eines Gasflusses zum Benutzer anzupassen.

5. Das System nach einem der Ansprüche 1 bis 4, wobei die Blutsauerstoffsättigungsdaten zudem repräsentativ für die Werte des sauerstoffhaltigen Hämoglobins sind, wobei der digitale Datenprozessor (106) zudem so programmiert ist, dass er relative Schwankungen der Werte des sauerstoffhaltigen Hämoglobins überwacht und auswertet.

## Revendications

1. Un système (100) de surveillance de l'hyperoxie chez un utilisateur, le système (100) comprenant :
une sonde de spectroscopie optique (104) pouvant être fixée sur la peau de l'utilisateur et permettant d'obtenir des données d'oxygénation du sang représentatives des niveaux de désoxyhémoglobine au fil du temps, dans lequel ladite sonde de spectroscopie optique (104) comprend une sonde de spectroscopie fonctionnant dans le proche infrarouge et dans lequel l'utilisateur est exposé à des pressions partielles d'oxygène s'écartant d'une valeur standard d'environ 0,21 atm dans des conditions standard de température et de pression ou est exposé à une autre pression que 1 atm ;
une interface utilisateur numérique (110) permettant d'afficher un indicateur de risque pour la santé de l'utilisateur ; et
un processeur de données numériques (106) connecté de manière opérationnelle à ladite sonde de spectroscopie optique (104) et à l'interface utilisateur (110), et programmé pour :
surveiller les variations desdits niveaux de désoxyhémoglobine ;
évaluer automatiquement lesdites variations des niveaux de désoxyhémoglobine par rapport aux variations préétablies des niveaux de désoxyhémoglobine faisant partie des profils d'oxygénation sanguine de référence, lesdits profils étant associés numériquement à un indice d'oxygénation sanguine préétabli définissant au moins un moindre niveau de risque d'hyperoxie pour la santé et un niveau de risque accru d'hyperoxie pour la santé ; et
émettre un signal représentatif dudit niveau de risque accru d'hyperoxie pour la santé en réponse à ladite évaluation en vue de l'affichage via ladite interface utilisateur numérique (110).

2. Le système de la revendication 1, permettant de surveiller un utilisateur exposé à des pressions supérieures à 1 atm.

3. Le système de l'une des revendications 1 à 2, permettant de surveiller un utilisateur participant à une activité nécessitant d'utiliser un appareil de fourniture d'oxygène (102).

4. Le système de l'une des revendications 1 à 3, permettant de surveiller un utilisateur participant à une activité nécessitant d'utiliser un appareil de fourniture d'oxygène (102), dans lequel l'appareil de fourniture d'oxygène comprend un recycleur et dans lequel ledit processeur de données numériques (106) est relié de manière opérationnelle à un contrôleur de recycleur pour ajuster automatiquement une sortie de recycleur, sous la forme d'un débit de gaz vers l'utilisateur, en réponse audit niveau de risque accru pour la santé.

5. Le système de l'une des revendications 1 à 4, dans lequel lesdites données d'oxygénation du sang sont en outre représentatives des niveaux d'hémoglobine oxygénée et dans lequel le processeur de données numériques (106) est en outre programmé pour surveiller et évaluer les variations relatives des niveaux d'hémoglobine oxygénée.
